# EUROPEAN PATENT SPECIFICATION

(11) **EP 1 194 536 B1**
(45) Date of publication and mention of the grant of the patent: **24.12.2008**
(21) Application number: 00943493.7
(22) Date of filing: 11.07.2000
(51) Int. Cl.: C12N 15/00, A01K 67/027, C12N 5/06, C12N 5/08

(54) **NON-HUMAN GENETICALLY MODIFIED MAMMAL LACKING THE ALPHA-FETOPROTEIN**
GENETISCH MODIFIZIERTES NICHTHUMANES TIER MIT MANGEL IN ALPHA-FETOPROTEIN
MAMMIFERE NON HUMAIN GENETIQUEMENT MODIFIE PRESENTANT UNE CARENCE D'ALPHA-FOETOPROTEINE

(30) Priority: 12.07.1999 US 143269 P
(43) Date of publication of application: 10.04.2002
(60) Divisional application: 08169064.6
(73) Proprietor: UNIVERSITE LIBRE DE BRUXELLES, B-1050 Bruxelles (BE)
(72) Inventor: GABANT, Philippe, B-1210 Brussels (BE); ROSCAM-SZPIRER, Josiane, B-1410 Waterloo (BE)
(74) Representative: Van Malderen, Joëlle
(86) International application number: PCT/BE2000/000081
(87) International publication number: WO 2001/003501

(56) References cited:
- WO-A-00/40693
- WO-A-00/53759
- WO-A-96/22787
- JIN D.K. ET AL: ". alpha.- Fetoprotein gene sequences mediating Afr2 regulation during liver regeneration." PROCEEDINGS OF THE NATIONAL ACADEMY OF SCIENCES OF THE UNITED STATES OF AMERICA, (21 JUL 1998) 95/15 (8767-8772)., XP000964629
- CIRILLO L.A. ET AL: "Developmental regulation of. alpha.- fetoprotein expression in intestinal epithelial cells of transgenic mice." DEVELOPMENTAL BIOLOGY, (1995) 168/2 (395-405)., XP000964762
- TYNER A.L. ET AL: "The ontogeny of. alpha.- fetoprotein gene expression i the mouse gastrointestinal tract." JOURNAL OF CELL BIOLOGY, (1990) 110/4 (915-927)., XP000964763
- MILLONIG J H ET AL: "Molecular analysis of the distal enhancer of the mouse alpha - fetoprotein gene." MOLECULAR AND CELLULAR BIOLOGY, (1995 JUL) 15 (7) 3848-56., XP000964767
- H. CHEN ET AL.: "Identification of a cis-acting element in the rat alpha-fetoprotein gene and its specific binding proteins in F9 cells during retinoic acid-induced differentiation." JOURNAL OF CELLULAR BIOCHEMISTRY, vol. 75, 1 January 1999 (1999-01-01), pages 25-34, XP000960630
- BUTTERFIELD L.H. ET AL: "Generation of human T-cell responses to an HLA-A2.1-restricted peptide epitope derived from. alpha.- fetoprotein." CANCER RESEARCH, (1 JUL 1999) 59/13 (3134-3142)., XP000960637
- HENRIETTE M.-F. ET AL: "Negative regulation of the.alpha.-foetoprotein gene in fibroblasts: Identification and characterization of cis and trans elements." FOLIA BIOLOGICA, (1997) 43/1 (5-13)., XP000964925
- YOUNG P R ET AL: "Construction and expression in vivo of an internally deleted mouse alpha - fetoprotein gene: presence of a transcribed Alu-like repeat within the first intervening sequence." NUCLEIC ACIDS RESEARCH, (1982 MAY 25) 10 (10) 3099-116., XP000964915
- SCOHY S. ET AL: "Identification of an enhancer and an alternative promoter in the first intron of the. alpha.- fetoprotein gene." NUCLEIC ACIDS RESEARCH, (1 OCT 2000) 28/19 (3743-3751)., XP000960628

## Description

### Field of the invention

The present invention is related to a non-human genetically modified mammal, preferably a knock-out mouse, comprising a partial or total deletion of a genetic sequence encoding the alpha-fetoprotein (AFP) and used as a model for the study of fertilization or contraceptive methods and drugs.

The present invention is related to a non-human mammalian pluripotential embryonic stem cell comprising a partial or total deletion of a genetic sequence encoding a mammal alpha-fetoprotein (AFP).

The present invention is also related to study, testing and/or screening method and device of known or unknown compounds able to bind the mammal alpha-fetoprotein (AFP) and that may be used as agonist or antagonist of oestrogens to the mammal alpha-fetoprotein.

### Background of the invention

Alpha-fetoprotein (AFP) is a glycoprotein present in the serum and a classical oncofetal marker. This protein is expressed at high levels during fetal life in the liver and the visceral endoderm of the yolk sac, and at lower levels in the developing gastrointestinal tract (Andrews et al., 1982; Tilghman and Belayew 1982), in the adult serum only trace amount are detected (Tilghman and Belayew 1982). The protein expressed by the embryos is secreted and present in the maternal blood circulation during gestation, the level of AFP concentration in the maternal serum is use to detect fetuses with spina bifida or Down's syndrome. The reason for this altered AFP level associated with those pathologies are not understood, but they have been used extensively in prenatal screening. The synthesis of AFP decreases dramatically after birth and only trace amounts are detected in adult liver. However expression of *afp* is associated with hepatocarcinomas and liver regeneration induced by partial hepatectomy or acute tetrachloride (CC14) intoxication. The control of *afp* gene expression has thus attracted much attention and it has been shown that *afp* expression is regulated by transcriptional mechanisms involving a large promoter and three distant enhancers (review of Chen et al. (1997)). Because AFP is synthesized during the G1 and S phases, it has been hypothesized that intracellular AFP affects cell growth (Leffert and Sell, 1974; Sell et al., 1975; Tsukada and Hirai, 1975; Belanger et al., 1978). The observation that AFP is able to bind estrogen led to the suggestion that AFP plays a role in the control of cell metabolism. In addition to binding estrogen, AFP, like albumin to which it is evolutionary related, is able to bind other steroids and endogenous and exogenous substances such as fatty acids, billirubin and various pharmaceutical agents suggesting that AFP may play a general transportation function. For the fetus, in this respect, AFP could serve as a modulator/modifier of various cell growth regulatory pathways during embryonic and fetal development in vertebrates by interacting and/or binding cytoplasmic chaperone proteins that normally escort nuclear receptors or transcription co-factors through the cytoplasm towards organelle interfaces (Mizejewski, 1995, 185) . AFP has also been proposed to protect the embryo against the maternal immune system, on the basis of the observation that addition of purified AFP into the culture of splenic or lymphnode mononuclear cells exerts a suppressive effect on antibody synthesis.

The different hypotheses proposed for AFP function(s) can be focused on the fetal life (stage at which the gene is strongly transcripted) since the protein is described as a fetoprotein.
Jin et al PNAS USA 1998, volume 95 p.8767, 8772 describes the construction of transgenic mice in which part of the alpha-fetoprotein (AFP gene) has been deleted. This document does not teach or suggest the phenotype of genetically modified female mammal comprising an homozygous mutation partial or total deletion in the genetic sequence encoding the wild type mammal alpha-fetoprotein (AFP).
At the present time, no document of the state of the art has suggested that the alpha-fetoprotein may play an essential role for female reproduction and fertility.

### Aims of the invention

The present invention aims to provide new models (animal models) as well as new methods and devices for the study, the testing and/or the screening of fertilization or contraceptive methods, compounds and compositions intended for adult mammals (including humans) and/or for the study, the testing and/or the screening of new methods, compounds or compositions intended for the treatment and/or the prevention of osteoporosis.

### Summary of the invention

The present invention is related to a non-human genetically modified sterile female mammal comprising an homozygous mutation partial or total deletion in a genetic sequence encoding the wild type mammal alpha-fetoprotein (AFP) preferably, this non human mammal is a mouse, preferably an amenorrheic mammal or a mammal that does not allow uteral nidification of an embryo.
The present invention is also related to the use of the non human mammal according to the invention for the study, the testing and other screening of anti-osteoporosis increasing fertility and/or contraceptive methods, compounds and compositions and for the study, the testing and the screening of oestrogens agonist or antagonist compounds to the mammal alpha-fetoprotein.

### Detailed description of the present invention

### Generation of mice carrying a germ-line mutation in the AFP gene

A clone containing a 129 genomic fragment of AFP loci was isolated from a lambda library. The library was screened with a probe containing the mouse afp promoter. The genomic insert of about 16 kb was subcloned in pKIL-PCR2 (Gabant et al., 1997). The targeting vector (pAFP K.O-1), consist of two recombination arms. The 5' arms (2.5 kb) was generated by polymerase chain reaction (PCR) using the following primers: N-Mer1: agagcggccgcggaagtgacaaagcagaacc annealing to the MerI sequence of the afp enhancer 1 (Godboute et al. (1988)) and a primer of the X-exon1: agactcgagggatgagggaagcgggtgtg complementary to the afp exon1. The PCR fragment generated using Pfu polymerase (Stratagene) was cloned in the pCR-blunt vector (Invitrogen).

The 3' arms were subcloned from the lambda into pBSIIKS+ vector (Stratagene). The 5' recombination arm was introduced upstream the 3' recombination arm. The IRES lacZ/neo reporter-selective cassette was introduced between these recombination arms. The tk2 negative selective marker was introduced into the SalI site to generate pAFP KO-1. This construction was linearized with NotI and electroporated into E14 ES cells. Correctly targeted clones were identified by Southern blot analysis using an external probe from the 5' region.

### ES cell injections and animal genotyping

Recombinant ES cells carrying the targeted allele were injected in C57BL/6J blastocysts. Animals were genotyped by extraction of DNA from tails.

### RNA isolation, Northern blot analysis

Total RNA was isolated using Trizol (Gibco BRL) extraction according to the manufacturer instructions. For the Northern analysis 20µg of total RNA were electrophoresed and transferred to nylon membranes as described. Filters were then hybridized.

### Western blot analysis

Proteins were separated by SDS-PAGE using 7.5% polyacrylamide gels in a Bio-Rad Mini Protean gel chamber and blotted onto Nitrocellulose filters in a Bio-Rad Trans Blot chamber according to the manufacturer's instructions. Proteins were detected using anti-AFP, anti-Albumin; anti Betagalactosidase serum (ICN Biochemicals) the signal was detected with ECL detection system (Amersham).

### LacZ reporter gene expression

To isolate embryonic stages, natural matings were set up and presence of a vaginal plug at noon the following day was taken as 0.5 days of gestation. Staged embryos were stained with X-Gal as wholemounts as described by Forrester et al. (1996). For cryostat sectioning, tissues were embedded in optimal cutting temperature (OTC) compounds (Miles, Inc., Elkart, IN), and sections stained for X-Gal were counterstained with haematoxylin and eosin, and mounted.

### Targeted mutagenesis of the afp gene

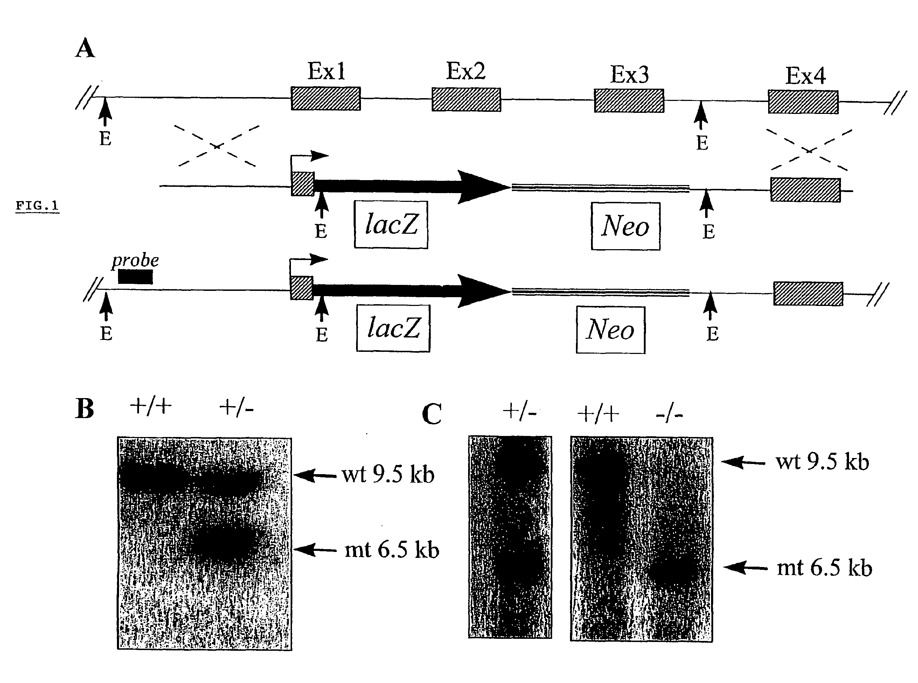

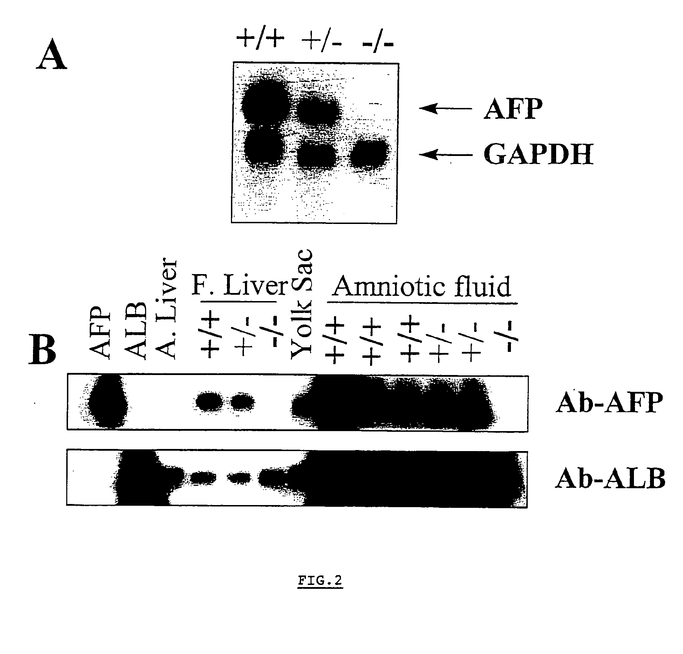

The *afp* gene was disrupted by gene targeting in embryonic stem (ES) cells. The *lacZ* reporter was introduced in *afp* gene by homologous recombination and placed under the control of the AFP promoter-enhancer region. The resulting allele is deleted for most of the sequence of exon1, for exon2 and 3 (see figure 1A) and homologous insertion was detected by Southern analysis (see figure 1B). To test the functionality of the reporter one may take advantage of the observation that AFP is expressed in embryoid bodies (Abe et al., 1996). Reporter gene activity is highly turn on in some cells of these bodies (see figure 2A). No expression of the reporter was detected in undifferentiated ES cells grown in the presence of LIF.

ES cells *afp* lacZ1/+ were injected into C57BL/6J blastocysts. Chimeric animals were obtained and mated with outbred CD1 or inbred 129/CGR to test for germ line transmission. Phenotypically normal heterozygous mice *afp* lacZ1/+ were generated and detected by Southern blot (see figure 1C).

### Reporter expression analysis

Expression of the lacZ reporter gene expression in embryonic and adult tissues was analyzed. As shown in Figure 2 the β-galactosidase activity was detected in predicted embryonic tissues. In the visceral endoderm only patches of cells were observed to turn the reporter strongly on. In the adult tissues tested specific staining was only detected in cells of the liver and in cells of the gut.

### Animals without AFP are viable

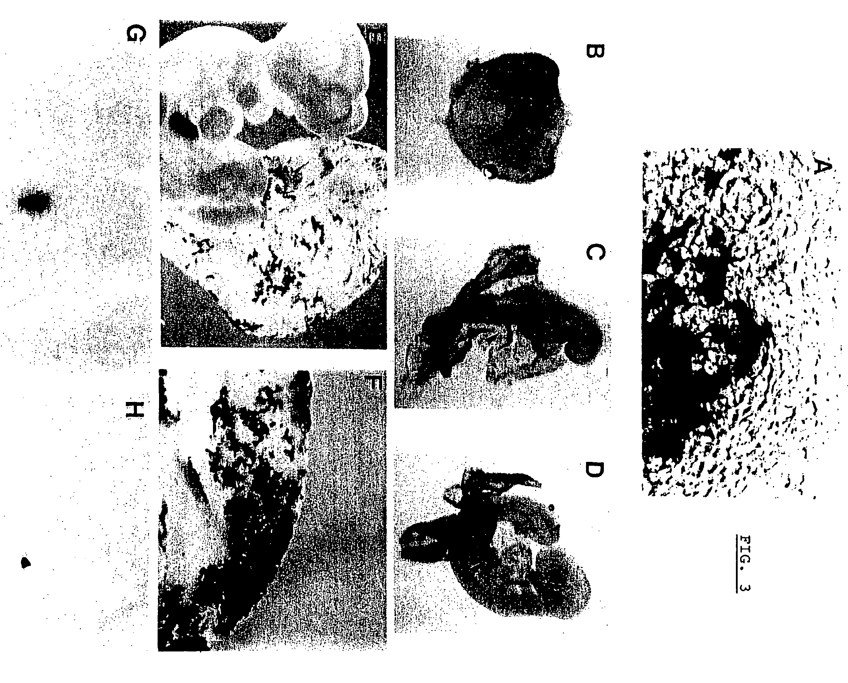

Intercrosses of heterozygotes (*afp*^{lacZ1/+}) gave rise to viable, apparently normal homozygous mutant mice at a Mendelian ratio in CD1 and C57/B16. On the other hand, a significant divergence was observed in the 129 background (Table 1). To determine whether the targeted allele indeed results in a null mutation, total RNA from the liver of embryos was analyzed by Northern blot hybridization (Figure 3A). A strong signal at 2.2kb corresponding to the *afp* transcript was detected in wild type and heterozygous embryos. No signal was detected in RNA samples extracted from homozygous embryonic liver, showing that the recombination disrupted the *afp* transcript in these animals. A Western blot was also performed on embryonic liver and amniotic fluid protein extracts (Figure 3B) and a strong signal was detected with the wild type extracts while no band corresponding to AFP was visible in the homozygous extracts demonstrating that these animals do not express AFP.

**Table 1: Intercrosses**

| **Strains** | **Parents** | | **offsprings** | | |
|---|---|---|---|---|---|
| | **Male** | **Female** | **+/+** | **+/-** | **-/-** |
| **CD1** | +/- | +/- | 108 | 258 | 102 |
| **129** | +/- | +/- | 34 | 48 | 13 |
| **C57/black-6** | +/- | +/- | 19 | 43 | 19 |

### AFP is required for female fertility

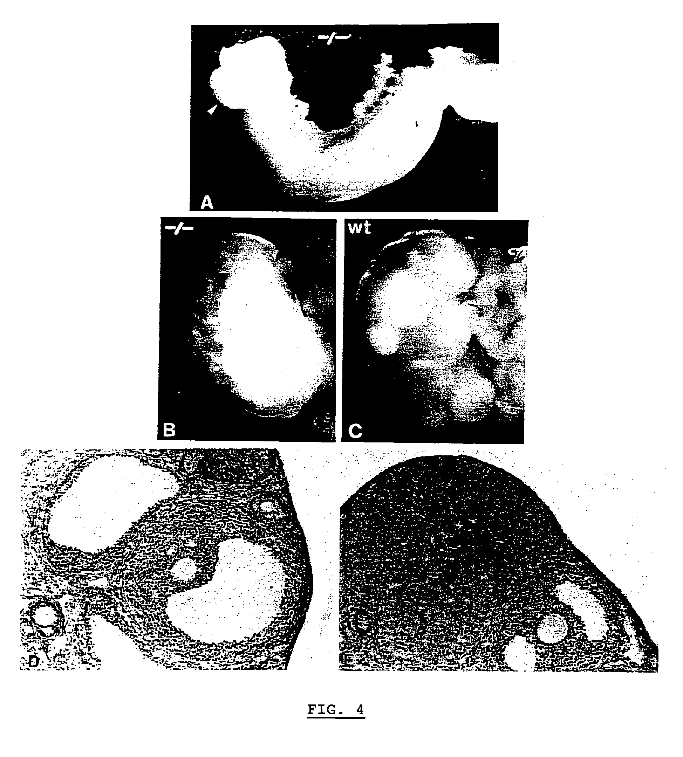

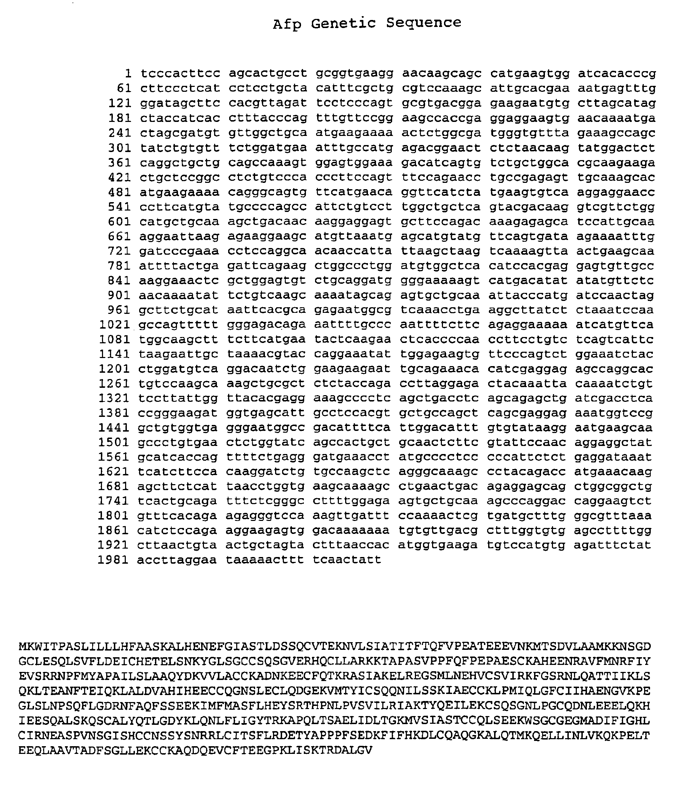

The Mendelian ratio obtained in CD1 and C57/black-6 background demonstrates that there is no reduction in the intercrosses. On the other hand, the divergence observed in 129 background suggests that AFP is involved in the gestation and that its importance is only revealed in some genetic contexts. In these litters derived from intercrossing heterozygous animals, homozygous embryos develop in the presence of their wild type and heterozygote littermates. AFP produced by these embryos is secreted and present in the maternal serum. To determine whether *afp* ^{lacZ1/lacZ1} mice are able to develop in the complete absence of AFP, *afp* ^{lacZ1/lacZ1} males and *afp* ^{lacZ1/lacZ1} females were mated. No pups were obtained from these intercrosses suggesting an essential role of AFP for development and/or fertility (see Table 2). To test if fertility was affected, *afp* ^{lacZ1/lacZ1} males and females were mated with wild type animals. Males homozygous for an *afp* disrupted allele appeared fertile and sired offspring but homozygous females never produce any live offspring. To test if natural matings occur with the *afp* ^{lacZ1/lacZ1} females leave with wild type males the vaginal plug were checked. No plugs were detected with those females, showing that the origin of the observed infertility is due to an absence of mating. To identify the defect underlying the reproductive capacity of homozygous females, the reproductive system of those animals was analyzed. The reproductive system of the *afp* ^{lacZ1/lacZ1} females was dissected and at this stage of the analysis appeared complete. A major anatomical difference is notable between ovaries from *afp* ^{lacZ1/lacZ1} and *afp* ^{+/+} : the ovaries of *afp* ^{lacZ1/lacZ1} are smooth, this observation suggests that those females do not ovulate. Histological analysis of mature *afp* ^{lacZ1/lacZ1} ovaries shown that their homozygous tissues do not contain corpus lutea, the lack of these structure is indicative of the absence of ovulation (see Figure 4). The *afp* ^{lacZ1/lacZ1} ovaries contain follicles at the different stages of maturation, this suggests that the default of AFP during the development has no effect on the female gametogenesis. However, the presence of follicles does not prove that these gametes are competent for maturation. To test the competence of the *afp* ^{lacZ1/lacZ1} follicles, they were dissected out and analyzed their potential of maturation in vitro. Complete maturation was obtained *in vitro* with the dissected oocytes from *afp* ^{lacZ1/lacZ1} animals. Taken together these data indicates that those females do not ovulate properly and thus that a signal needed to trigger ovulation is absent in the *afp* ^{lacZ1/lacZ1} mice.

**Table 2: Phenotypical analysis**

| **Parents** | | **offsprings** | | |
|---|---|---|---|---|
| **Male** | **Female** | +/+ | +/- | -/- |
| -/- | -/- (2) | 0 | 0 | 0 |
| -/- | +/+ (6) | 0 | 71 | 0 |
| +/+ | -/- (13) | 0 | 0 | 0 |

Table 2: Infertility phenotype of the *_{afp}*^{lacZ1/lacZ1} (-/-) homozygous animals were mated, no offsprings were obtained from these matings. To test fertility of the *afp*^{lacZ1/lacZ1} (-/-) males and females, homozygous males and females were mated with wild type (+/+) animals. For the different breedings the number of mating is given in brackets.

It was also observed that *afp*^{lacZ1/lacZ1} follicles are able to mature normally *in vitro* (data not shown) suggesting that the defect could be in a signal required to trigger ovulation and indeed ovulation can be induced in these animals by a superovulation protocol (Table 3).

**Table 3: Ovulation induction in afp^{lacZ1/+} and afp^{lacZ1/lacZ1} females**

| **Mice injected** | **Number of oocytes obtained** |
|---|---|
| afp^{lacZ1/+} females (9 weeks) | 37 |
| afp^{lacZ1/lacZ1} females (9 weeks) | 31 |

Table 3: Induction of ovulation in *afp*^{lacZ1/lacZ1} females. *afp*^{lacZ1/lacZ1} and *afp*^{lacZ1/+} females were hormonally treated to induce ovulation. The average number of postovulation oocytes obtained from 6 individual females tested.

### Mouse genotyping by PCR

In order to identify a possible mutation or deletion in the afp gene, a specific genotyping of afp *-*/mice by PCR has been developed. Two primers are used for the first afp#1 anneals in the *afp* promoter region (-116 bp to -137 bp): according to the +1 of the mouse *afp* gene). The second primer afp#2 is complementary to the first exon of the mouse *afp* gene (+141 bp to +160 bp: according to the +1 of the mouse *afp* gene): sequence deleted in the *afp* -/knock-out mouse described in the text.
Sequence afp#1: cccctgctctgttaattattg
Sequence afp#2: gaaaatagctcccaagtcac

No amplification product (300 bp) was observed in *afp* -/- animals. This amplification is present in wild type and heterozygous mice.

To differentiate +/- from wild type, a second PCR is performed using two primers giving an amplification on the DNA introduced in the genome of the transgenic animals (this sequence is not present in wild type animals).

For the knock-out mouse for *afp* described in the text: a couple of primers complementary to *lacZ* (*E. coli* gene) can be used.
lacz#1: acaacgtcgtgactgggaaaac
lacZ#2: taatgggataggttacgt

Those primers will only give a signal (287 bp) on +/- and no signal on wild type DNA samples.

The physiological role of the alpha-fetoprotein, the most abundant serum protein expressed by mammalian embryos remains to be establish. This protein related to albumin excreted by the embryos into the maternal blood circulation has attracted attention and due to its abundance it has been postulated that the presence of this protein was essential for embryonic development. To determine the function of AFP this gene was disrupted by homologous recombination in embryonic stem cells. Surprisingly homozygous *afp*^{lacZ1/lacZ1} are viable showing that expression of AFP by the embryo itself is not require for normal and complete development. This phenotype shows that the structure of the homozygous females was generally maintained. The different stages of ovocytes maturation are founded in these ovaries and shown to accomplish their maturation *in vitro.* Ovulation was induced in these females by hormonal induction and ovocytes were produced. Although AFP is synthesized at a high level during fetal life (mainly by the liver and the visceral endoderm of the yolk sac), low level of AFP mRNA has been reported in different other fetal and adult tissues as well as in adult rats. However, the level of expression in such tissues is very low. Another explanation is that the ovulation in those females is affected by the lack of AFP during development.

The fact that in adults afp^{lacZ1/lacZ1} ovulation can be induced argues for the absence in those females of a signal needed to trigger ovulation. This suggests that AFP is involved in the transportation of an element needed for ovulation in the adults.

By the disruption of the mouse AFP, one may show that fetal serum protein is required for females ovulation.

The fact that analbuminic rats are fertile shows that at least albumin cannot rescue AFP. This show that albumin and AFP plays two different role and that AFP is involved in the function of females ovaries.

The specific phenotype of the non-human mammals according to the invention is also illustrated in the enclosed figure 4, which presents the anatomical and histological analysis for the afp^{lacZ1/lacZ1} ovaries:
A: Structure of the ovary (arrow) and uterus of an adult *afp*^{lacZ1/lacZ1} (-/-) female;
B: Ovary from 12 weeks old *afp*^{lacZ1/lacZ1};
C: Ovary from a 12 weeks old wild type female: the surface distortions are due to the presence of large type follicles, whereas *afp*^{lacZ1/lacZ1}ovaries are smooth;
D: The general histological structure of the *afp*^{lacZ1/lacZ1} ovaries is not affected and mature Graafian follicles are present in those tissues (section from a fourth month old female);
E: At 4 months, the wild type ovaries exhibit large corpus lutea, indicative of successful ovulation. Those structures are never found in *afp*^{lacZ1/lacZ1} ovaries.

Therefore, the non-human female mammals that present homozygously said mutation, partial or total deletion in the afp gene do not cycle. The surface of the ovaries is smooth and is not characterized by the presence of large follicles. Their histological structure is not generally affected, and Graafian follicles are identified in the tissues but no large corpus lutea is present.

Furthermore, females comprising an homozygous mutation or partial or total deletion in the *afp* gene do not allow uterus nidification of an embryo.

Additional experiments have shown that the *afp* gene is not essential for the survival of the mice. Indeed, as females give birth to severa animals, it was possible that the *afp^{-l-}* may survive to brothers and sisters (*afp*^{+/+} or *afp*^{*+*/*-*}) simultaneously present in the uterus of the female.

Therefore, in order to extrapolate the observed phenotype and genotype to the human population, the inventors have shown that blastocysts implanted one by one in pseudogravite females will obtain the birth of alive *afp*^{-/-} animals that present the same phenotype as above-described (fertile males, sterile females).

Therefore, the *afp*^{-/-} phenotype corresponds to alive sterile females, which is a phenotype that may exist in the mouse population as well as in the human population.

### REFERENCES

- Andrews G.K. et al., J. Biol. Chem. 257 pp. 5148-5153 (1982)
- Tilghman S.M. and Belayew A., Proc. Natl. Acad. Sci. USA 79 pp. 5254-5257 (1982)
- Godbout R. et al., Mol. Cell. Biol. 6 pp. 477-487 (1986)
- Godbout R. et al., Mol. Cell. Biol. 8(3) pp. 1169-1178 (1998)
- Chen H. et al., Critical Rev. In Eucaryotic Gene Expression 7 pp. 11-41 (1997)
- Festin et al., Biochem. & Biophys. Acta 24789 pp. 307-314 (1999)
- Leffert H. L. and Sell S., J. Cell Biol. 61 pp. 823-829 (1974)
- Sell S. et al., Ann. NY Acad. Sci. 259 pp. 45-58 (1975)
- Tsukada Y. and Hirai H., Ann. NY Acad. Sci. 259 pp. 37-44 (1975)
- Belanger L. et al., Scand. J. Immunol. 8 (Suppl 8) pp. 239-246 (1978)
- Mizejewski G.J. New insights into AFP structure and function: potential biomedical applications. In Mizejewski G.J., Porter IH (eds). Alpha-Fetoprotein and congenital disorder. Orlando :Academic Press :5-34 (1985)
- Mizejewski G.J., Life Sci. 56 pp. 1-9 (1995)
- Abe K. et al., Exp. Cell Res. 25 pp. 27-34 (1996)

## Claims

1. A non-human genetically modified sterile female mammal comprising a homozygous mutation, partial or total deletion in a genetic sequence encoding the wild type mammal alpha-fetoprotein (AFP).

2. The non-human mammal according to claim 1, **characterized in that** it is a mouse.

3. The non-human mammal according to the claim 1 or 2 **characterized in that** it is an amenorrheic mammal.

4. The non-human mammal according to the claim 1 to 3 **characterized in that** it does not allow an uteral nidification of an embryo.

5. Use of the non-human mammal according to any one of the preceding claims, for the study, the testing and/or the screening of anti-osteoporosis, increasing fertility and/or contraceptive methods, compounds and compositions and for the study, the testing and the screening of oestrogens agonist or antagonist compounds to the mammal alpha-fetoprotein.

6. A mouse pluripotential embryonic stem cell carrying an allele comprising a partial or total deletion of a genetic sequence encoding an alpha-fetoprotein (AFP).

7. A mouse pluripotential embryonic stem cell comprising a total deletion of a genetic sequence encoding an alpha-fetoprotein (AFP).

## Patentansprüche

1. Nicht-menschliches genetisch modifiziertes steriles weibliches Säugetier, das eine homozygote Mutation, eine teilweise oder vollständige Deletion in einer genetischen Sequenz, die das Wildtyp-Säugetier-Alphafetoprotein (AFP) codiert, umfasst.

2. Nicht-menschliches Säugetier nach Anspruch 1, **dadurch gekennzeichnet, dass** es eine Maus ist.

3. Nicht-menschliches Säugetier nach Anspruch 1 oder 2, **dadurch gekennzeichnet, dass** es ein amenorrhoisches Säugetier ist.

4. Nicht-menschliches Säugetier nach Anspruch 1 bis 3, **dadurch gekennzeichnet, dass** es keine Gebärmuttereinnistung eines Embryos ermöglicht.

5. Verwendung des nicht-menschlichen Säugetiers nach einem der vorhergehenden Ansprüche, für die Untersuchung, das Testen und/oder das Prüfen von Anti-Osteoporose-, Fruchtbarkeitssteigerungs- und/oder Empfängnisverhütungsverfahren, -verbindungen und -zusammensetzungen und für die Untersuchung, das Testen und das Prüfen von Östrogenen, die Agonisten- oder Antagonistenverbindungen für das Säugetier-Alphafetoprotein sind.

6. Pluripotente embryonale Mausstammzelle, die ein Allel trägt, das eine teilweise oder vollständige Deletion einer genetischen Sequenz, die ein Alphafetoprotein (AFP) codiert, umfasst.

7. Pluripotente embryonale Mausstammzelle, die eine vollständige Deletion einer genetischen Sequenz, die ein Alphafetoprotein (AFP) codiert, umfasst.

## Revendications

1. Mammifère femelle stérile non humain génétiquement modifié comprenant une mutation homozygote, délétion partielle ou totale dans une séquence génétique codant pour l'alpha-foetoprotéine (AFP) mammalienne de type sauvage.

2. Mammifère non humain selon la revendication 1, **caractérisé en ce qu'**il est une souris.

3. Mammifère non humain selon la revendication 1 ou 2, **caractérisé en ce qu'**il est un mammifère aménorrhéique.

4. Mammifère non humain selon la revendication 1 à 3, **caractérisé en ce qu'**il ne permet pas une nidation utérine d'un embryon.

5. Utilisation du mammifère non humain selon l'une quelconque des revendications précédentes, pour étudier, tester et/ou cribler des procédés, composés et compositions anti-ostéoporose, améliorant la fécondité et/ou contraceptifs, et pour étudier, tester et cribler de composés agonistes ou antagonistes d'oestrogènes pour l'alpha-foetoprotéine mammalienne.

6. Cellule souche embryonnaire pluripotente murine portant un allèle comprenant une délétion partielle ou totale d'une séquence génétique codant pour une alpha-foetoprotéine (AFP).

7. Cellule souche embryonnaire pluripotente murine comprenant une délétion totale d'une séquence génétique codant pour une alpha-foetoprotéine (AFP).
